# EUROPEAN PATENT APPLICATION

(11) **EP 4 741 496 A1**
(43) Date of publication of application: **13.05.2026**
(21) Application number: 24835479.7
(22) Date of filing: 19.08.2024
(51) Int. Cl.: C12N 5/074, C12N 15/85, C12N 15/12, C12N 5/10

(54) **METHOD FOR REPROGRAMMING HUMAN SOMATIC CELLS INTO INDUCED PLURIPOTENT STEM CELLS**

(30) Priority: 04.07.2023 CN 202310811030
(71) Applicant: Exoneuglia Biotechnology (Suzhou) Co., Ltd., Suzhou, Jiangsu 215127 (CN)
(72) Inventor: GUO, Ying, Suzhou, Jiangsu 215127 (CN); YANG, Yixing, Suzhou, Jiangsu 215127 (CN)
(74) Representative: AWA Denmark A/S
(86) International application number: PCT/CN2024/113016
(87) International publication number: WO 2025/008001

(57) **Abstract**

A simpler and more efficient method for reprogramming human somatic cells into induced pluripotent stem cells. According to the method, three transcription factors of OCT4, SOX2, and LIN28 are simultaneously transferred into somatic cells, and then reprogramming is performed under the co-induction of four small molecules of Peficitinib, VTP50469, SB203580, and FPFT-2216, wherein the peripheral blood reprogramming efficiency thereof can reach 0.02%, the induction period is only 14-16 days, and the system is stable.

## Description

### TECHNICAL FIELD

The present invention relates to the technical field of pluripotent stem cell reprogramming, specifically to a simpler and more efficient method for reprogramming human somatic cells into induced pluripotent stem cells.

### BACKGROUND ART

Since Shinya Yamanaka's research group first induced mouse fibroblasts into pluripotent stem cells in 2006, the technology of pluripotent stem cell reprogramming has undergone rapid development. The reprogramming of human somatic cells has also progressively been achieved. For example, Junying Yu's research group has reprogrammed peripheral blood cells into induced pluripotent stem cells by introducing six transcription factors: OCT4, SOX2, LIN28, NANOG, C-MYC and KLF4. The research groups of Miguel Esteban and Ruiqing Pei successfully reprogrammed renal epithelial cells into induced pluripotent stem cells by introducing three transcription factors (OCT4, SOX2, and KLF4) and microRNA302/367 into urinary cells. In 2022, Hongkui Deng's research group successfully reprogrammed human-derived fibroblasts into pluripotent stem cells using a complete small-molecule reprogramming approach; however, the induction process was extremely time-consuming.

The process of reprogramming of somatic cells into induced pluripotent stem cells involves drastic transitions in both cell fate and characteristic genes expression. Currently, scientists have a general understanding of the genes that are completely suppressed and that are completely activated following somatic-to-stem cell conversion. By introducing small molecules that promote pathways for target gene activation and inhibit pathways for silencing undesirable genes, it is possible to achieve an efficient and stable reprogramming scheme with minimal transcription factors. However, research progress in reprogramming with minimal transcription factors currently remains slow. Of the few schemes that have been reported, issues such as poor reproducibility and inconsistent efficiency still exist. For example, Chinese invention patent (Publication No. CN114645023B), titled "System and method of reprogramming peripheral blood mononuclear cells into induced pluripotent stem cells", has a reprogramming efficiency of only about 0.006%. Furthermore, the number of clones varies significantly in multiple replicate experiments, with efficiency fluctuating between 0.002% and 0.006%, indicating substantial instability. Therefore, to gain deeper insights into the roles of various transcription factors in reprogramming somatic cells into embryonic stem cells, and to improve reprogramming schemes for somatic cell-to-embryonic stem cell conversion, it is necessary to develop a highly efficient and reproducible reprogramming scheme that is particularly suitable for difficult-to-transfect cell types, such as peripheral blood cells.

### SUMMARY

Based on this, one objective of the present invention is to provide a method of reprogramming human somatic cells into induced pluripotent stem cells, comprising the following steps: introducing only three reprogramming induction factors OCT4, SOX2, and LIN28 into somatic cells, and
culturing the somatic cells in the presence of chemical inducers to obtain induced pluripotent stem cells;
wherein the chemical inducers consist of Peficitinib, VTP50469, SB203580, and FPFT-2216; and wherein a concentration of Peficitinib is 0.5 µM to 1.5 µM, a concentration of VTP50469 is 0.1 µM to 1 µM, a concentration of SB203580 is 1 µM to 3 µM, and a concentration of FPFT-2216 is 1 µM to 5 µM.

In a preferred embodiment, the three reprogramming induction factors OCT4, SOX2, and LIN28 are introduced into the somatic cells in the form of nucleic acids thereof or protein products thereof.

In a preferred embodiment, the three reprogramming induction factors OCT4, SOX2, and LIN28 are introduced into the somatic cells in the form of DNA or mRNA thereof.

In a preferred embodiment, the form of introducing the three reprogramming induction factors OCT4, SOX2, and LIN28 into somatic cells includes, but is not limited to, a form of Sendai virus transfection system, an mRNA transfection system, or an episomal plasmid transfection.

In a preferred embodiment, the somatic cells are hematopoietic precursor cells derived from peripheral blood.

In a preferred embodiment, the method of reprogramming human somatic cells into induced pluripotent stem cells comprises the following steps:
introducing the three transcription factors OCT4, SOX2, and LIN28 into somatic cells; inducing the somatic cells by using an induction medium containing the chemical inducers of claim 1; and finally culturing the somatic cells by using a pluripotent stem cell medium until the induced pluripotent stem cell clones are fully mature, followed by randomly picking clones for expansion;
wherein, the induction medium is a pluripotent stem cell medium supplemented with 2 µM to 4 µM chir99021, 80 ng/ml to 120 ng/ml FGF2, 200 µM to 300 µM NaB, 1 µM to 3 µM SB203580, 0.5 µM to 1.5 µM peficitinib, 0.05 µM to 0.2 µM VTP50469, 1 µM to 5 µM FPFT-2216, 1% to 3% (v/v) B27, and 1% (v/v) N2; and wherein the pluripotent stem cell medium is mTeSR1 medium.

Specifically, the method comprises introducing the three transcription factors OCT4, SOX2, and LIN28 into somatic cells via electroporation of episomal plasmid, wherein the episomal plasmid is a modified pCEP4 vector carrying the three transcription factors, and wherein the two transcription factors OCT4 and SOX2 are constructed on the same pCEP4 vector.

Specifically, the method comprises: immediately adding peripheral blood expansion medium after electroporation, seeding the cells in a plate coated with basement membrane matrix; and culturing for 46 h to 50 h; adding an induction medium and culturing for another 46 h to 50 h; subsequently performing partial medium changes with fresh induction medium and continuing induction for 46 h to 50 h, repeated 1 to 3 times; completely removing all the old medium and adding pluripotent stem cell medium for culture; replacing the pluripotent stem cell medium daily until the induced pluripotent stem cell clones are fully mature; and picking clones for expansion at any time.

Specifically, the peripheral blood expansion medium is a serum-free medium supplemented with 80 ng/mL to 120 ng/mL SCF, 80 ng/mL to 120 ng/mL FLT-3, 10 ng/mL to 30 ng/mL IL-3, 10 ng/mL to 30 ng/mL IL-6, and 80 ng/mL to 120 ng/mL TPO.

The present invention reprograms human peripheral blood cells into induced pluripotent stem cells using three transcription factors and four small molecule inhibitors. The reprogramming efficiency can reach 0.02%, which is about 3.3-fold higher than that of the prior art (patent No. CN114645023B), the induction period is only 14 to 16 days, and the system demonstrates more stable.

### BRIEF DESCRIPTION OF THE DRAWINGS

FIG. 1 shows microscopic observations during the induction process of Treatment Group 1;
FIG. 2 shows a comparison of statistical results of clone numbers obtained in different induction treatment groups in Example 1;
FIG. 3 shows the results of flow cytometry analysis of pluripotent cell markers expressed by pluripotent stem cells obtained in Treatment Group 1;
FIG. 4 shows immunofluorescence detection results DAPI staining fluorescence detection results, and Merge results of markers for pluripotent stem cells obtained in Treatment Group 1,
FIG. 5 shows purity analysis results of induced pluripotent stem cells obtained in Treatment Group 1;
FIG. 6 shows tumor formation experiment results in mice using induced pluripotent stem cells obtained in Treatment Group 1.

### DETAILED DESCRIPTION

The present invention will be further described in detail below with reference to specific examples to enable those skilled in the art to understand the invention more clearly. The following examples are provided solely for illustrative purposes and are not intended to limit the scope of the present invention. Based on the specific examples of the present invention, any other examples that can be obtained by ordinary technical personnel in the art, without involving creative labor, shall fall within the protection scope of the present invention.

Unless otherwise stated, reagents used in the examples of the present invention are commercially available products. The experimental methods not explicitly detailed in the following examples are typically conducted under conventional conditions, such as the conditions described in Sambrook et al., Molecular Cloning: A Laboratory Manual (New York: Cold Spring Harbor Laboratory Press, 1989), or the conditions recommended by the manufacturer.

In the present invention, using peripheral blood cells as an example, the signaling pathways originally characterizing their hematopoietic cell fate are silenced upon conversion into stem cells. These pathways include the JAK-STAT signaling pathway, the AKT signaling pathway, the IKZF1 gene pathway, the PTPN22 gene pathway, and the TAL1 gene pathway. By optimizing the reprogramming induction factors to the three factors OCT4, SOX2, and LIN28, while simultaneously conducting induction culture with four chemical small molecules, Peficitinib, VTP50469, SB203580, and FPFT-2216, the present invention achieves the conversion of peripheral blood cells into induced pluripotent stem cells. Particularly for hematopoietic precursor cells derived from peripheral blood, the reprogramming efficiency can reach 0.02%, the induction period is only 14 to 16 days, and the system is more stable.

The three induction factors optimized and identified in the present invention, OCT4, SOX2, and LIN28, are all previously reported induction factors. Regarding the sequence information, the NCBI accession number of the OCT4 transcription factor is 5460, the NCBI accession number of the SOX2 transcription factor is 6657, and the NCBI accession number of the LIN28 transcription factor is 79727.

The chemical inducer "Peficitinib" described in the present invention is an inhibitor of Janus kinase (JAK), purchased from MCE (Cat. No. HY-19568).

The chemical inducer "VTP50469" described in the present invention is an inhibitor of Menin-MLL interaction, purchased from MCE (Cat. No. HY-114162).

The chemical inducer "SB203580" described in the present invention is a p38 MAPK inhibitor, purchased from MCE (Cat. No. HY-10256).

The chemical inducer "FPFT-2216" described in the present invention is a zinc finger transcription factor IKZF1 inhibitor, purchased from MCE (Cat. No. HY-145319).

In the present invention, SCF is a stem cell factor, FLT-3 is a type III receptor tyrosine kinase, IL-3 is interleukin-3, IL-6 is interleukin-6, and TPO is thyroidperoxidase.

StemPro^{™}-34 Medium is purchased from Gibco (Cat. No. 10640-019).

In the present invention, chir99021 is a glycogen synthase kinase 3 (GSK-3) inhibitor, FGF2 is fibroblast growth factor 2, NaB is sodium butyrate, which is the sodium salt form of butyrate and is a histone deacetylase (HDAC) inhibitor, B27 is a serum-free supplement, and N-2 is a supplement.

mTeSR1 medium is purchased from Stemcell (Cat. No. 85850).

The chemical inducer "Tofacitinib" in the comparative examples of the present invention is a JAK3 inhibitor, purchased from MCE (Cat. No. HY-40354).

In the present invention, the form for introducing the three reprogramming induction factors OCT4, SOX2, and LIN28 into somatic cells includes, but is not limited to, the form of nucleic acid, such as DNA or mRNA, or the form of protein products thereof.

In the present invention, the form for introducing the three reprogramming induction factors OCT4, SOX2, and LIN28 into somatic cells includes, but is not limited to, a form of Sendai virus transfection system, an mRNA transfection system, or an episomal plasmid transfection.

### Example 1 Comparison of Reprogramming Under Conditions of Different Inducing Factor

The inducing factors were introduced via electroporation of episomal plasmids in Treatment Groups 1 to 6. The groups differed only in the chemical inducer small molecules added to the pluripotent stem cell induction medium. The specific steps were as follows:

### (1) Construction of Episomal Plasmids:

Construction of pCEP4-OCT4-IRES-SOX2 plasmid: The empty vector plasmid pCEP4 (Cat. No. V04450) was purchased from the Thermo website. The gene sequences for OCT4 and SOX2 were subsequently downloaded from the NCBI website. The NCBI accession number of the OCT4 transcription factor gene sequence is 5460, and the NCBI accession number of the SOX2 transcription factor gene sequence is 6657. The target genes (transcription factor gene sequences) were ligated into the empty vector plasmid pCEP4 between the SnaB I and Asc I restriction sites using a homologous recombination method.

Construction of pCEP4-Lin28 plasmid: The empty vector plasmid pCEP4 (Cat. No. V04450) was purchased from the Thermo website. The gene sequence for Lin28 was subsequently downloaded from the NCBI website. The NCBI accession number of the Lin28 transcription factor gene sequence is 79727. The target gene (Lin28 transcription factor gene sequence) was ligated into the empty vector plasmid pCEP4 between the SnaB I and Asc I restriction sites using a homologous recombination method.
(2) Expansion of Peripheral Blood Cells: Freshly isolated peripheral blood cells (peripheral blood cells obtained with ethical approval were purchased from Shanghai HYCELLs Company) were seeded into a 6-well plate at a density of 5×10⁶ cells/mL and cultured statically for 7 days at 37°C in an incubator. The peripheral blood cell expansion medium used was StemPro^{™}-34 medium supplemented with SCF 100 ng/mL, FLT-3 100 ng/mL, IL-3 20 ng/mL, IL-6 20 ng/mL, and TPO 100 ng/mL.
(3) Electroporation: Prior to electroporation, 500 µL of diluted basement membrane matrix (Matrigel) was added to a 12-well plate and placed in a CO₂ incubator for coating. The expanded cells were collected and washed. After counting, the peripheral blood mononuclear cells (PBMCs) were placed in a 1.5 mL centrifuge tube at a density of 2×10⁶ cells/mL. The tube was loaded into a centrifuge and centrifuged at 200 g for 5 minutes. After centrifugation, the supernatant was aspirated with a pipette and set aside for later use.

According to the Thermo Neon electroporation instrument protocols, buffer was added. The cell pellet was mixed with 5 µg of pCEP4-OCT4-IRES-SOX2 plasmid and 2 µg of pCEP4-Lin28 plasmid, and the mixture was loaded into the electroporation instrument for electroporation. Peripheral blood expansion medium was immediately added thereafter, and the cells were seeded in the coated 6-well plate at a density of 5×10⁵ cells/mL and cultured statically for 48 h at 37°C in a CO₂ incubator.

(4) Induction: At 48 h post-electroporation, 1 mL of pluripotent stem cell induction medium was directly added to the original wells, and the culture was continued statically for 48 h at 37°C in a CO₂ incubator; On day 4 post-electroporation, half of the old medium in the original wells was aspirated and discarded, 1 mL of fresh pluripotent stem cell induction medium was then added, and the culture was continued statically for 48 h at 37°C in a CO₂ incubator; On day 6 post-electroporation, all the old medium in the original wells was aspirated and discarded, 1 mL of fresh pluripotent stem cell induction medium was added, and the culture was continued statically for 48 h at 37°C in a CO₂ incubator; On day 8 post-electroporation, all the old medium in the original wells was aspirated and discarded, 1 mL of fresh pluripotent stem cell induction medium was added, and the culture was continued statically for 48 h at 37°C in a CO₂ incubator; On day 10 post-electroporation, all the old medium in the original wells was aspirated and discarded, 1 mL of fresh mTeSR1 medium was added, and the culture was continued statically at 37°C in a CO₂ incubator; The mTeSR1 medium was changed daily until the induced pluripotent stem cells were fully mature, approximately on day 14 or 15 post-electroporation. Clones were then picked for expansion.

The pluripotent stem cell induction medium for Treatment Group 1 was mTeSR1 medium supplemented with 3 µM chir99021, 100 ng/mL FGF2, 250 µM NaB, 2 µM SB203580, 1 µM peficitinib, 1 µM FPFT-2216, 0.1 µM VTP50469, 2% (v/v) B27, and 1% (v/v) N-2.

The pluripotent stem cell induction medium for Treatment Group 2 was mTeSR1 medium supplemented with 3 µM chir99021, 100 ng/mL FGF2, 250 µM NaB, 1 µM peficitinib, 2% (v/v) B27, and 1% (v/v) N-2.

The pluripotent stem cell induction medium for Treatment Group 3 was mTeSR1 medium supplemented with 3 µM chir99021, 100 ng/mL FGF2, 250 µM NaB, 2 µM Tofacitinib, 2% (v/v) B27, and 1% (v/v) N-2.

The pluripotent stem cell induction medium for Treatment Group 4 was mTeSR1 medium supplemented with 3 µM chir99021, 100 ng/mL FGF2, 250 µM NaB, 2 µM SB203580, 2% (v/v) B27, and 1% (v/v) N-2.

The pluripotent stem cell induction medium for Treatment Group 5 was mTeSR1 medium supplemented with 3 µM chir99021, 100 ng/mL FGF2, 250 µM NaB, 2 µM SB203580, 1 µM peficitinib, 1 µM FPFT-2216, 2% (v/v) B27, and 1% (v/v) N-2.

No inducing small molecules were added in Treatment Group 6.

The numbers of induced pluripotent stem cell clones in each treatment group were counted and compared.

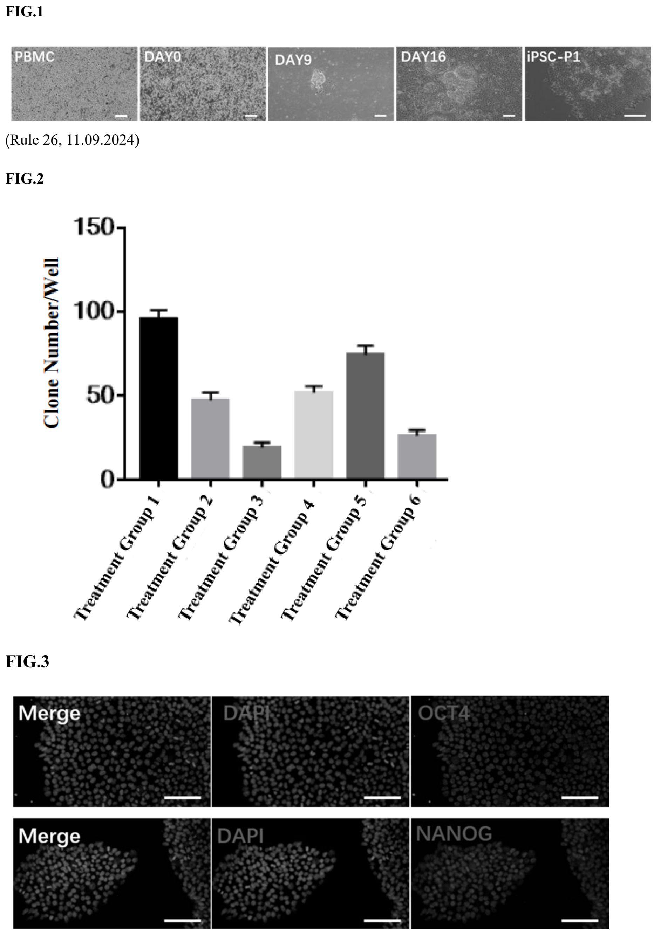

Alkaline Phosphatase Staining Experiment: The Alkaline Phosphatase Staining Kit (Cat. No. 40749ES) was purchased from Yeasen Biotechnology. Day 14 cell samples from Treatment Groups 1 to 6 were stained according to the instruction. Due to the high expression of alkaline phosphatase in induced pluripotent stem cells, purple-coloured induced pluripotent stem cell clones were observed in the wells. Subsequently, ImageJ software was employed to quantify the clones, specifically the number of clones per 5.0×10⁵ reprogrammed PBMCs. The results are shown in Table 1 and FIG. 2 below.

**Table 1 Comparison of Programming Efficacy of Different Reprogramming Methods**

| | Inducer Small Molecules | Average Number of Pluripotent Stem Cell Clone/Well | | |
|---|---|---|---|---|
| | | Experiment 1 | Experiment 2 | Experiment 3 |
| Treatment Group 1 | Peficitinib+VTP50469+ SB203580+FPFT-2216 | 90 | 95 | 101 |
| Treatment Group 2 | Peficitinib | 43 | 52 | 47 |
| Treatment Group 3 | Tofacitinib | 20 | 16 | 22 |
| Treatment Group 4 | SB203580 | 51 | 56 | 48 |
| Treatment Group 5 | Peficitinib+SB203580+ FPFT-2216 | 80 | 74 | 69 |
| Treatment Group 6 | No inducer | 25 | 24 | 30 |

As can be seen from Table 1, using Treatment Group 1 for reprogramming induction can yield a greater number of pluripotent stem cell clones, with a reprogramming efficiency of 0.02%. The system demonstrated high stability and reproducibility. Observation results of human peripheral blood mononuclear cells (PBMCs) in Treatment Group 1 under an electron microscope prior to treatment, post-treatment, and on day 9 and 16 post-treatment are shown in FIG. 1.

### Example 2 Identification of Induced Pluripotent Stem Cells Obtained in Treatment Group 1

The induced pluripotent stem cells obtained in Treatment Group 1 were characterized and identified using various methods including flow cytometry, immunofluorescence, and quantitative PCR.

Immunofluorescence analysis of markers OCT4 and NANOG was performed on the induced pluripotent stem cells obtained in Treatment Group 1. The results are shown in FIG. 3. The first row shows the immunofluorescence results, DAPI staining fluorescence detection results, and Merge results for the OCT4 marker. The second row shows the immunofluorescence results, DAPI staining fluorescence detection results, and Merge results for the NANOG marker.

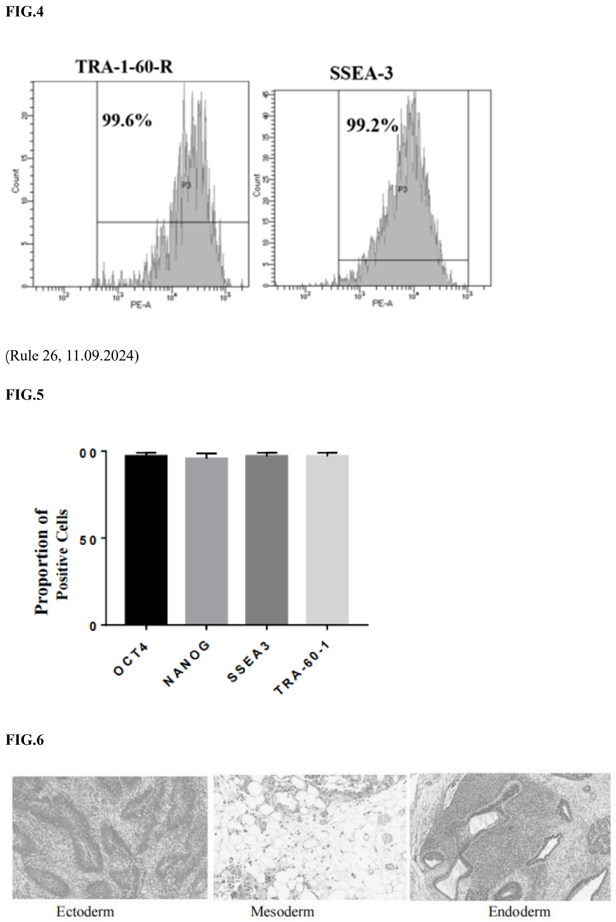

Flow cytometry analysis of the obtained induced pluripotent stem cells was performed for the following molecular markers: TRA-1-60 and SSEA-3. The results are shown in FIG. 4, demonstrating that the obtained cells possess the characteristics of pluripotent stem cells.

### Example 3 Purity Analysis of Induced Pluripotent Stem Cells Obtained in Treatment Group 1

Based on the immunofluorescence and flow cytometry results in Example 2, statistical analysis of the data using ImageJ software showed that the proportion of cells positive for pluripotency markers (OCT4/NANOG/TRA-1-60/SSEA-3) all exceeded 95%, as shown in FIG. 5.

### Example 4 Mouse Tumor Formation Experiment

5×10⁶ iPSC-P15 cells (i.e., passage 15 cells) obtained in Treatment Group 1 were taken and resuspended in a 1:1 mixture of 50 µL DMEMF12 and 50 µL Matrigel matrix gel, then kept on ice. Subsequently, the cell mixture was injected subcutaneously into the left upper armpit of an immunodeficient mouse using an insulin syringe needle. The cells were allowed to expand in vivo for 8-12 weeks. When a distinct tumor-like protrusion developed under the armpit, it was removed for paraffin sectioning and H&E staining to observe three germ layers differentiation.

As can be seen from FIG. 6, the iPS cells differentiated into three germ layers in vivo, forming a complete teratoma, proving their pluripotent differentiation ability.

It should be noted that the above examples are solely intended to further elucidate and describe the technical solutions of the present invention and do not constitute further limitations on the technical solutions of the present invention. The methods of the present invention are merely preferred embodiments and are not intended to limit the protection scope of the present invention. Any modifications, equivalent substitutions, improvements, or other adaptations, made within the spirit and principles of the present invention shall be encompassed within the protection scope of the present invention.

## Claims

1. A method of reprogramming human somatic cells into induced pluripotent stem cells, **characterized by** comprising the following steps:
introducing only three reprogramming induction factors, OCT4, SOX2, and LIN28, into the somatic cells, and
culturing in the presence of chemical inducers to obtain induced pluripotent stem cells;
wherein the chemical inducers consist of Peficitinib, VTP50469, SB203580, and FPFT-2216;
and wherein a concentration of Peficitinib is 0.5 µM to 1.5 µM, a concentration of VTP50469 is 0.1 µM to 1 µM, a concentration of SB203580 is 1 µM to 3 µM, and a concentration of FPFT-2216 is 1 µM to 5 µM.

2. The method of reprogramming human somatic cells into induced pluripotent stem cells according to claim 1, **characterized in that** the three reprogramming induction factors OCT4, SOX2, and LIN28 are introduced into the somatic cells in the form of nucleic acids thereof or protein products thereof.

3. The method of reprogramming human somatic cells into induced pluripotent stem cells according to claim 1, **characterized in that** the three reprogramming induction factors OCT4, SOX2, and LIN28 are introduced into the somatic cells in the form of DNA or mRNA thereof.

4. The method of reprogramming human somatic cells into induced pluripotent stem cells according to claim 1, **characterized in that** the form for introducing the three reprogramming induction factors OCT4, SOX2, and LIN28 into somatic cells includes, but is not limited to, a form of Sendai virus transfection system, an mRNA transfection system, or an episomal plasmid transfection.

5. The method of reprogramming human somatic cells into induced pluripotent stem cells according to claim 1, **characterized in that** the somatic cells are hematopoietic precursor cells derived from peripheral blood.

6. The method of reprogramming human somatic cells into induced pluripotent stem cells according to any one of claims 1 to 5, **characterized by** comprising the following steps:
introducing the three transcription factors OCT4, SOX2, and LIN28 into somatic cells;
inducing by using an induction medium containing the chemical inducers of claim 1; and
finally culturing by using a pluripotent stem cell medium until the induced pluripotent stem cell clones are fully mature, followed by randomly picking clones for expansion;
wherein, the induction medium is a pluripotent stem cell medium supplemented with 2 µM to 4 µM chir99021, 80 ng/ml to 120 ng/ml FGF2, 200 µM to 300 µM NaB, 1 µM to 3 µM SB203580, 0.5 µM to 1.5 µM peficitinib, 0.05 µM to 0.2 µM VTP50469, 1 µM to 5 µM FPFT-2216, 1% to 3% (v/v) B27, and 1% (v/v) N2; wherein the pluripotent stem cell medium is mTeSR1 medium.

7. The method of reprogramming human somatic cells into induced pluripotent stem cells according to claim 6, **characterized by** comprising introducing the three transcription factors OCT4, SOX2, and LIN28 into somatic cells via electroporation of episomal plasmid, wherein the episomal plasmid is a modified pCEP4 vector carrying the three transcription factors, and wherein the two transcription factors OCT4 and SOX2 are constructed on the same pCEP4 vector.

8. The method of reprogramming human somatic cells into induced pluripotent stem cells according to claim 7, **characterized by** comprising:
immediately adding peripheral blood expansion medium after electroporation, seeding the cells in a plate coated with basement membrane matrix, and culturing for 46 h to 50 h;
adding an induction medium and culturing for another 46 h to 50 h;
subsequently performing partial medium changes with fresh induction medium and continuing induction for 46 h to 50 h, repeated 1 to 3 times;
completely removing all the old medium and adding pluripotent stem cell medium for culture;
replacing the pluripotent stem cell medium daily until the induced pluripotent stem cell clones are fully mature; and
picking clones for expansion at any time.

9. The method of reprogramming human somatic cells into induced pluripotent stem cells according to claim 8, **characterized in that** the peripheral blood expansion medium is a serum-free medium supplemented with 80 ng/mL to 120 ng/mL SCF, 80 ng/mL to 120 ng/mL FLT-3, 10 ng/mL to 30 ng/mL IL-3, 10 ng/mL to 30 ng/mL IL-6, and 80 ng/mL to 120 ng/mL TPO.
